# EUROPEAN PATENT APPLICATION

(11) **EP 2 071 041 A1**
(43) Date of publication of application: **17.06.2009**
(21) Application number: 08021467.9
(22) Date of filing: 10.12.2008
(51) Int. Cl.: C12Q 1/68

(54) **Nucleic acid detection probe**

(30) Priority: 13.12.2007 JP 2007322043; 24.10.2008 JP 2008274783
(71) Applicant: Hitachi High-Technologies Corporation, Minato-ku Tokyo 105-8717 (JP)
(72) Inventor: Uematsu, Chihiro, Tokyo 100-8220 (JP); Nakashima, Yukie, Tokyo 100-8220 (JP)
(74) Representative: Strehl Schübel-Hopf & Partner

(57) **Abstract**

It is intended to provide a nucleic acid detection probe that is designed with a high degree of flexibility. The present invention provides a nucleic acid detection probe used in nucleic acid detection, wherein the amount of a particular target gene present in a sample is examined by simultaneously hybridizing, to the target nucleic acid, a first probe labeled at one end with a fluorophore and a second probe labeled with a quencher at an end different from the labeled end of the first probe and observing quenching attributed to the interaction between the fluorophore and the quencher.

SEQUENCE LISTING

<110>  HITACHI HIGH-TECHNOLOGIES CORPORATION

<120>  Probe for nucleic acid detection

<150>  JP 2007-322043
<151>  2007-12-13

<150>  JP 2008-274783
<151>  2008-10-24

<160>  32

<170>  PatentIn version 3.4

<210>  1
<211>  28
<212>  DNA
<213>  Artificial

<220>
<223> Inventor: Nakashima, Yukie; Uematsu, Chihiro

<220>
<223>  Inner primer (forward) used in the present invention

<400>  1
tccaggggtc ttaacttgat ggaaaaat                                          28

<210>  2
<211>  15
<212>  DNA
<213>  Artificial

<220>
<223>  Outer primer (forward) used in the present invention

<400>  2
tccagaaacg tttcg                                                        15

<210> 3
<211> 27
<212> DNA
<213> Artificial

<220>
<223> Inner primer (reverse) used in the present invention

<400> 3
ggatccaggc ccagaaaaaa agactgt 27


<210> 4
<211> 16
<212> DNA
<213> Artificial

<220>
<223> Outer primer (reverse) used in the present invention

<400> 4
agggcttggt caatat 16


<210> 5
<211> 35
<212> DNA
<213> Artificial

<220>
<223> First detection prove used in the present invention

<400> 5
ggtttctcag gaagcaaaaa acttggcctt cctgg 35


<210> 6
<211> 35
<212> DNA
<213> Artificial

<220>
<223> Second detection prove used in the present invention

<400> 6
tccagggggt gcttacaatc ctgatgtttt cattc 35

<210> 7
<211> 27
<212> DNA
<213> Artificial

<220>
<223> Primer (forward) used in the present invention

<400> 7
gtcccattat tttccagaaa cgtttcg 27

<210> 8
<211> 25
<212> DNA
<213> Artificial

<220>
<223> Primer (reverse) used in the present invention

<400> 8
aagtacttca gggcttggtc aatat 25

<210> 9
<211> 36
<212> DNA
<213> Artificial

<220>
<223> First detection prove used in the present invention

<400> 9
ggtttctcag gaagcaaaaa acttggcctt acctgg 36

<210> 10
<211> 35
<212> DNA
<213> Artificial

<220>
<223>  Second detection prove used in the present invention

<400>  10
tccagggggt gcttacaatc ctgatgtttt cattc                                35


<210>  11
<211>  23
<212>  DNA
<213>  Artificial

<220>
<223>  Primer (forward) used in the present invention

<400>  11
gcagaaagcg tctagccatg gcg                                             23


<210>  12
<211>  23
<212>  DNA
<213>  Artificial

<220>
<223>  Primer (reverse) used in the present invention

<400>  12
catggtgcac ggtctacgag acc                                             23


<210>  13
<211>  20
<212>  DNA
<213>  Artificial

<220>
<223>  Reverse trascription primer used in the present invention

<400>  13
tgctcatggt gcacggtcta                                                 20

<210> 14
<211> 99
<212> DNA
<213> Artificial

<220>
<223> Detection probe used in the present invention

<400> 14
tgttgggtcg cgaaaggccc cttctcactg ttctctcata gaagtgatga gggaacagag 60

cactcatctc ttctccctgt tagactgcta gccgagtag 99


<210> 15
<211> 18
<212> DNA
<213> Artificial

<220>
<223> 3'-terminal sequence that hybridizes with HCV gene

<400> 15
gatgagccga tcgtcaga 18


<210> 16
<211> 19
<212> DNA
<213> Artificial

<220>
<223> 5'-terminal sequence that hybridizes with HCV gene

<400> 16
tgttgggtcg cgaaaggcc 19


<210> 17
<211> 19
<212> DNA
<213> Artificial

<220>

<223>  Primer (forward) used in the present invention

<400>  17
tctttccaag caacagcag                                                    19


<210>  18
<211>  21
<212>  DNA
<213>  Artificial

<220>
<223>  Primer (reverse) used in the present invention

<400>  18
cgtttgctga tgaactaagt c                                                 21


<210>  19
<211>  21
<212>  DNA
<213>  Artificial

<220>
<223>  First detection prove used in the present invention

<400>  19
caatgaccaa atcaaagaaa t                                                 21


<210>  20
<211>  21
<212>  DNA
<213>  Artificial

<220>
<223>  First detection prove used in the present invention

<400>  20
caatgaccaa atcaaagaaa t                                                 21


<210>  21
<211>  21

<212>  DNA
<213>  Artificial

<220>
<223>  Second detection prove used in the present invention

<400>  21
actcgcaagg ttagtgctga g                                                21


<210>  22
<211>  21
<212>  DNA
<213>  Artificial

<220>
<223>  Second detection prove used in the present invention

<400>  22
actcgcaagg ttagtgctga g                                                21


<210>  23
<211>  29
<212>  DNA
<213>  Artificial

<220>
<223>  Primer (sense) used in the present invention

<400>  23
tgctatgtca cttccccttg gttctctca                                        29


<210>  24
<211>  29
<212>  DNA
<213>  Artificial

<220>
<223>  Primer (antisense) used in the present invention

<400>  24
agtgggggga catcaagcag ccatgcaaa                                        29

<210>  25
<211>  19
<212>  DNA
<213>  Artificial

<220>
<223>  First detection prove used in the present invention


<220>
<221>  misc_feature
<222>  (18)..(18)
<223>  n represents inosine

<400>  25
ctgcagaatg ggacaggnt                                                    19


<210>  26
<211>  18
<212>  DNA
<213>  Artificial

<220>
<223>  First detection prove used in the present invention

<400>  26
gaggaagctg cagaatgg                                                     18


<210>  27
<211>  18
<212>  DNA
<213>  Artificial

<220>
<223>  First detection prove used in the present invention

<400>  27
aagataccat caatgagg                                                     18

<210> 28
<211> 16
<212> DNA
<213> Artificial

<220>
<223> First detection prove used in the present invention

<400> 28
cagaatggga tagggt 16


<210> 29
<211> 17
<212> DNA
<213> Artificial

<220>
<223> Second detection prove used in the present invention

<400> 29
acagccagta catgcag 17


<210> 30
<211> 18
<212> DNA
<213> Artificial

<220>
<223> Second detection prove used in the present invention

<400> 30
gatagattgc atcccagt 18


<210> 31
<211> 16
<212> DNA
<213> Artificial

<220>
<223> Second detection prove used in the present invention

<400> 31
aggctgcaga atggga 16

<210> 32
<211> 20
<212> DNA
<213> Artificial

<220>
<223> Second detection prove used in the present invention

<400> 32
acacccagta catgcagggc 20

## Description

### BACKGROUND OF THE INVENTION

### Field of the Invention

The present invention relates to a nucleic acid (DNA or RNA) detection method. More specifically, the present invention relates to a gene detection method comprising the step of specifically hybridizing an oligonucleotide probe to a sample (single-stranded or doublestranded nucleic acid such as genomic DNA or RNA or PCR products).

### Background Art

The analysis of gene expression levels with high sensitivity and wide dynamic range plays an exceedingly important role in functional analysis of genes or in disease study or diagnosis. For example, the test of infection such as hepatitis, HIV, tuberculosis germs, or sexually transmitted infection requires conducing gene quantification on an infectious pathogen at the initial stage for circumventing infection spread or effectively treating the infection. Alternatively, pharmaceutical fields require conducting quantification on a gene that differs in a disease-specific manner, for identifying a target in drug discovery or evaluating the effects of a drug.

Such gene quantification generally involves detecting the gene of interest after amplification by a method such as PCR (Polymerase Chain Reaction), NASBA (Nucleic Acid Sequence Based Amplification), or LAMP (Loop mediated isothermal amplification). A probe used in the detection is, for example, a TaqMan, Molecular Beacon, or Quenching probe. Of them, the detection using the TaqMan probe requires using an enzyme having 5'→3' exonuclease activity. Therefore, this TaqMan probe is applicable to a PCR amplification method using DNA polymerase having 5'→3' exonuclease activity and however, cannot be used in NASBA or LAMP, which is an isothermal amplification method that requires using DNA or RNA polymerase having strand displacement activity but free from 5'→3' exonuclease activity. Thus, detection probes that can be used in LAMP or NASBA are Molecular Beacon and Quenching probes.

The Molecular Beacon probe is designed such that a sequence therein hybridizes to a target nucleic acid. In addition, this probe needs to be designed such that the stem sequence takes a stable hairpin structure in the absence of the target nucleic acid. This is because if the stem sequence fails to take the stable hairpin structure, incomplete quenching occurs, leading to increase in background. The prevention thereof requires designing, with care, the sequence that hybridizes to the target nucleic acid and the stem sequence. As a result, the probe sequence is disadvantageously designed with a limited degree of flexibility.

On the other hand, the Quenching probe is labeled at the 5' or 3' end with a fluorophore. This probe employs the phenomenon in which fluorescence is quenched through the interaction between the fluorophore in the probe bound with a target nucleic acid and guanine in the target nucleic acid sequence. Fluorescence is quenched in the presence of the target nucleic acid and is not quenched in the absence of the target nucleic acid. Therefore, the presence or absence of the target nucleic acid can be determined. However, the fluorophore that is quenched through its interaction with guanine is limited to Pacific Blue, TAMRA, CR6G, BODIPY FL, or the like and is not suitable for multicolor detection. Moreover, the probe must be designed such that the probe is located at a position that permits the interaction with guanine in the target nucleic acid sequence. Thus, the probe is also disadvantageously designed with a limited degree of flexibility.
R. K. Saiki, et al., Science, 1988, 239, 487-491
J. Compton, et.al., Nature, 1991, 350, 91-92
T. Notomi, et al., Nucleic Acids Research, 2000, 28, e63
Pamela M., et al., Proc. Natl. Acad. Sci., USA, August 1991, 88, 7276-7280
S. Tyagi, F. R. Kramer, Nature Biotechnology, 1996, 14, 303-308
S. Kurata, et al., Nucleic Acids Research 2001, 29, e34

### SUMMARY OF THE INVENTION

A main object of the present invention is to provide a probe that is designed with a high degree of flexibility to be applicable to detection by a nucleic acid amplification method using DNA polymerase free from 5'→3' exonuclease activity.

To attain the object, the present inventors have found a method wherein the presence or absence of a target nucleic acid is determined by hybridizing first and second probes labeled with a fluorophore and a quencher, respectively (and vice versa) to the target nucleic acid in tandem such that a fluorescent signal from the fluorophore is quenched.

The probes used in the present invention are a first probe which hybridizes to the target nucleic acid and a second probe which hybridizes thereto on the 3' end side of the first probe to give a gap of 0 to 10 bases, preferably 0 to 3 bases, most preferably 1 base, between the first and second probes. The first probe is labeled at the 3' end with a quencher. The second probe is labeled at the 5' end with a fluorophore and labeled with a phosphate group or an amino group at the 3' end to prevent the second probe from being extended.

When the target nucleic acid is absent in a reaction solution, the first and second probes are present each independently in the reaction solution from which fluorescence derived from the second probe is detected. On the other hand, when the target nucleic acid is present, the first and second probes hybridize to the target nucleic acid. In this case, the first and second probes hybridize thereto in tandem to give the gap. Therefore, the quencher as a label at the 3' end of the first probe and the fluorophore as a label at the 5' end of the second probe are located close to each other such that a fluorescent signal from the fluorophore is quenched through fluorescent resonance energy transfer.

In the course of nucleic acid amplification reaction, the target nucleic acids are formed as the reaction progresses. Therefore, the first and second probes gradually hybridize to the amplified target nucleic acids. Specifically, fluorescent signal intensity from the reaction solution is decreased as time passes. In case of a conventional Molecular Beacon or TaqMan probe, fluorescent signal intensity increases as the target nucleic acid is amplified. The timing when the fluorescent signal intensity exceeds a certain threshold value depends on the amount of the target nucleic acid before the initiation of reaction. On the other hand, in case of the nucleic acid detection probe of the present invention, fluorescent signal intensity decreases as the target nucleic acid is amplified. The timing when the fluorescent signal intensity falls below a certain threshold value depends on the amount of the target nucleic acid before the initiation of reaction. Thus, a calibration curve is prepared in the same way as in the conventional Molecular Beacon or TaqMan probe by plotting the amount of a fluorescent signal decreased, calculated from the absolute value of the fluorescent signal. Based on the calibration curve, the target nucleic acid can be quantified.

Alternatively, the first probe may be labeled at the 3' end with a fluorophore, and the second probe may be labeled at the 5' end with a quencher. In this case as well, the first and second probes hybridize to the target nucleic acid to give a gap of 0 to 10 bases, preferably 0 to 3 bases, most preferably 1 base, between the first and second probes such that a fluorescent signal from the fluorophore is quenched through the interaction between the fluorophore and the quencher. In this case as well, the second probe is labeled at the 3' end to prevent the second probe from being extended.

Alternatively, the first and second probes may be ligated as one probe. Specifically, a sequence portion corresponding to the first probe and a sequence portion corresponding to the second probe are designed to hybridize to the target nucleic acid in tandem. These sequence portions are ligated via a sequence portion which does not hybridize to the target nucleic acid to prepare a probe labeled at the 5' end with a fluorophore and labeled at the 3' end with a quencher. This sequence portion which does not hybridize thereto has a length that is equal to or larger than the total length of the sequence portion corresponding to the first probe and the sequence portion corresponding to the second probe and needs to be a length on the order of a few hundred bases. Preferably, the sequence portion is 50 to 200 bases in length. When the target nucleic acid is absent, the probe takes a linear form. Therefore, the fluorophore and the quencher as labels at the 5' and 3' ends, respectively, of the probe are kept apart in the reaction solution from which fluorescence is detected. When the target nucleic acid is present, the 5' and 3' ends of the probe hybridize to the target nucleic acid in tandem. As a result, the fluorophore and the quencher are located close to each other such that a fluorescent signal from the fluorophore is quenched through fluorescent resonance energy transfer. Thus, the state in which the target nucleic acid are amplified as the reaction progresses can be measured using even the first and second probes that are ligated, in the same way as in the use of the first and second probes described above. Alternatively, the probe may be labeled at the 3' end with a fluorophore and at the 5' end with a quencher.

Thus, according to the present invention, a probe can be designed without sequence limitations on a target sequence.

Moreover, the present invention provides a nucleic acid detection probe kit shown below.

In one embodiment, a kit comprises two oligonucleotide probes having sequences which respectively recognize a target nucleic acid and hybridize to the target nucleic acid in tandem to give a gap of 0 to 10 bases, preferably 0 to 3 bases, most preferably 1 base, between the probes. One of the oligonucleotide probes flanking this gap is labeled with a fluorophore, and the other oligonucleotide probe is labeled with a quencher.

In an alternative embodiment, the first and second probes are ligated as one oligonucleotide probe labeled at the 5' and 3' ends with a fluorophore and a quencher, respectively. This oligonucleotide probe hybridizes to the target nucleic acid in tandem such that the fluorophore and the quencher are located in proximity to each other.

In an alternative embodiment, an oligonucleotide probe may be modified at the end with a functional group such as an amino group or with biotin such that a user may label the oligonucleotide probe with the fluorophore or quencher. In this case, a kit can additionally comprise a fluorophore or quencher having a portion introduced for reaction with the functional group.

The kit of the present invention comprises the oligonucleotide probe(s) as an essential component and may also comprise other reagents necessary for detection reaction, for example, a reaction buffer, substrate nucleotides, amplification primers, and enzymes.

In the present invention, two or one oligonucleotide probe(s) labeled with a fluorophore and a quencher are allowed to act on a target gene, and changes in fluorescent signal generated through fluorescent resonance energy transfer are detected. As a result, a nucleic acid detection probe that is functionally similar to a conventional Molecular Beacon or Quenching probe hardly designed with a high degree of flexibility can be designed regardless of a target gene sequence.

### BRIEF DESCRIPTION OF THE DRAWINGS

FIG. 1 is a diagram showing a flow of target gene detection according to the present invention.
FIG. 2 is a diagram showing a flow of target gene detection according to the present invention.
FIG. 3 is a diagram showing a flow of target gene detection according to the present invention.
FIG. 4 is a graph showing fluorescent intensities before and after the amplification reaction of a target nucleic acid.
FIG. 5 is a graph showing the relationship of the number of gap bases between first and second probes with a quenching rate.
FIG. 6 is a graph showing the relationship of the difference of Tm values between first and second probes with a quenching rate.
FIG. 7 is a graph showing fluorescent signal intensities before and after amplification reaction.
FIG. 8 is a graph showing results of real-time detection through amplification reaction.
FIG. 9 is a graph showing fluorescent signal intensities before and after amplification reaction.
FIG. 10 is a graph showing fluorescent signal intensities before and after amplification reaction.
FIG. 11 is a graph showing results of real-time detection through amplification reaction.

### Description of Symbols

1, 11 ... First probe
2, 12 ... Second probe
3 ... Target nucleic acid
5 ... Fluorophore
6 ... Quencher
7 ... Phosphate group
8 ... Amino group
15 ... Linear oligonucleotide probe
20, 25, 30, 35, 40, 45, 50, 60, 61, 62, 63 ... Graph

### DETAILED DESCRIPTION OF THE PREFERRED EMBODIMENTS

Hereinafter, the present invention will be described specifically with reference to the drawings.

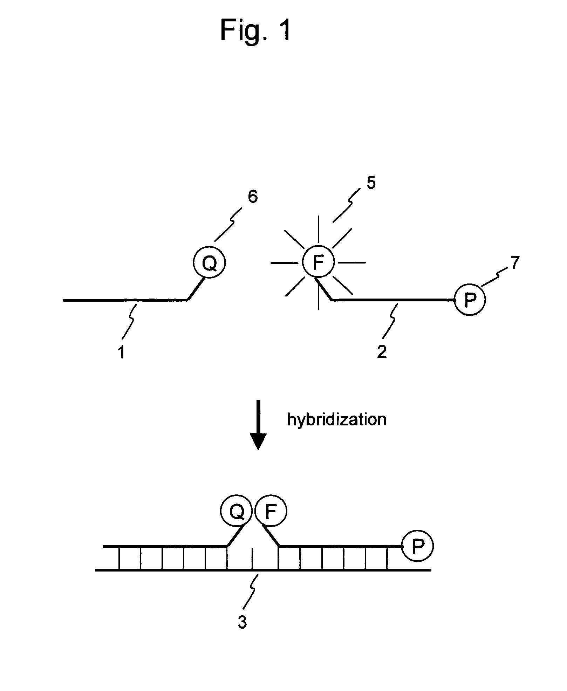

A first flow of the present invention is shown in FIG. 1. The present invention provides probes for detecting a nucleic acid sequence, which comprise a first probe 1 having a nucleotide sequence specific to the nucleotide sequence of a target nucleic acid 3 and a second probe 2 having a sequence that hybridizes to the target nucleic acid 3 such that the 5' end thereof is located 1 base apart from the 3' end of the first probe 1. The first probe 1 is labeled at the 3' end with a quencher 6 such as DABCYL or BHQ. The second probe 2 is labeled at the 5' end with a fluorophore 5 such as FAM or Cy5. Moreover, the second probe is phosphorylated or aminated or modified by dideoxy modification at the 3' end to prevent the probe from being extended (the second probe shown in FIG. 1 is labeled with phosphate 7). The first probe is labeled with one quencher, and the second probe is labeled with one fluorophore. Specifically, one fluorophore or quencher is used in labeling per probe, leading to easy probe synthesis and improved synthesis yields. According to the present invention, even a fluorophore never before used for a Molecular Beacon or TaqMan probe due to difficulty in double labeling can be used. For example, even a fluorophore such as Alexa 350, Alexa 750, or Alexa 790 can be used, which is excited in an ultraviolet region or emits fluorescence in a far infrared region. When the target nucleic acid 3 is present, the amount of the target nucleic acid 3 is increased with progress in, for example, PCR reaction as the reaction time passes. In this case, the first probe 1 and the second probe 2 hybridize to the amplification product of the target nucleic acid 3. The first probe 1 and the second probe 2 are designed to hybridize to the target nucleic acid in a positional relationship to give the gap of 1 base. Therefore, the quencher 6 as a label in the first probe 1 and the fluorophore 5 as a label in the second probe are located close to each other such that fluorescence from the fluorophore 5 is quenched. In FIG. 1, the first probe 1 and the second probe 2 hybridize to the target nucleic acid to give a gap of 1 base. However, the gap may be in the range of 0 (no gap) to 10 bases, preferably 0 to 3 bases.

In this context, examples of the fluorophore that can be used in the present invention can include FAM, TET, HEX, Fluorescein, FITC, CR6G, ROX, TAMRA, JOE, Texas Red, Oregon Green, Yakima Yellow, Pacific Blue, Cascade Blue, Cy Dye series, CAL Fluor series, ALEXA series, Rhodamine series, and BODIPY series. Examples of the quencher that can be used in the present invention can include TAMRA, Dabcyl, Eclipse(R) Dark Quencher, BHQ series, and DDQ series. Examples of possible combinations of the fluorophore and the quencher used in the present invention include, but not limited to, combinations of ALEXA 350 with BHQ-0, FAM with BHQ-1, ROX with BHQ-2, Cy5 with BHQ-3, TET with Dabcyl, Fluorescein with TAMRA, HEX with DDQ I, Rhodamine 6G with DDQ II, and Yakima Yellow with Eclipse(R) Dark Quencher.

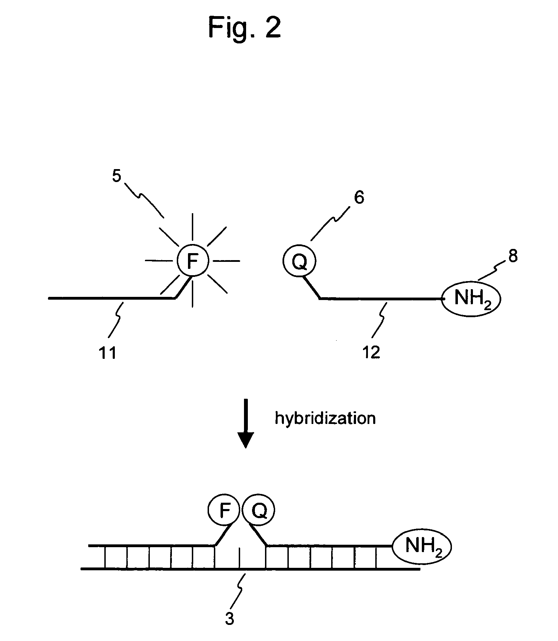

A second flow of the present invention is shown in FIG. 2. In this embodiment, a first probe 11 is labeled with a fluorophore 5, and a second probe 12 is labeled with a quencher 6. In FIG. 2, the second probe 12 is modified at the 3' end with an amino group 8 to prevent the second probe 12 from being extended. In this case as well, the first probe 11 and the second probe 12 hybridize to a target nucleic acid 3 in the same way as in FIG. 1. Thus, the fluorophore 5 and the quencher 6 are located close to each other to cause quenching effects.

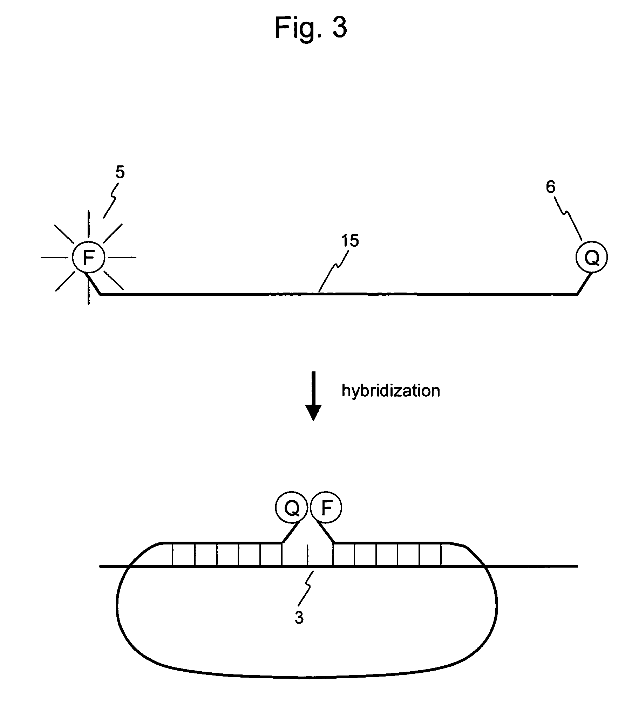

A third flow of the present invention is shown in FIG. 3. In this embodiment, the first and second probes are ligated. In this case, a linear oligonucleotide probe 15 used has a sequence portion corresponding to the first probe and a portion corresponding to the second probe that are designed to hybridize to the target nucleic acid in tandem and ligated via a third sequence portion which does not hybridize to the target nucleic acid. The oligonucleotide probe 15 is labeled at the 5' and 3' ends with a fluorophore 5 and a quencher 6, respectively. When the target nucleic acid 3 is absent, the oligonucleotide probe 15 takes linear conformation. Therefore, the fluorophore 5 and the quencher 6 as labels at the 5' and 3' ends, respectively, of the oligonucleotide probe 15 are kept apart in the reaction solution from which fluorescence is detected. On the other hand, when the target nucleic acid 3 is present, the 5' and 3' ends of the oligonucleotide probe 15 hybridize to the target nucleic acid 3 in tandem. As a result, the fluorophore 5 and the quencher 6 are located close to each other such that a fluorescent signal from the fluorophore is quenched through fluorescent resonance energy transfer. In this case as well, the probe may be labeled at the 3' end with a fluorophore and at the 5' end with a quencher.

Using the first and second probes of the present invention, the presence or absence of the target nucleic acid can be determined by measuring the fluorescent intensities of the solution before and after PCR amplification reaction. In this case, the Tm values of the first and second probes are set to temperatures about 5 to 10°C higher than those of primers. This is because fluorescence measurement is conducted during annealing, and thus, the probes must hybridize with reliability to the target nucleic acid before the primers do. For example, the Tm values of primers used in [Example 2] described later are 59.0°C (forward primer) and 57.2°C (reverse primer). By contrast, the Tm values of the probes are 65.3°C (first probe) and 66.3°C (second probe).

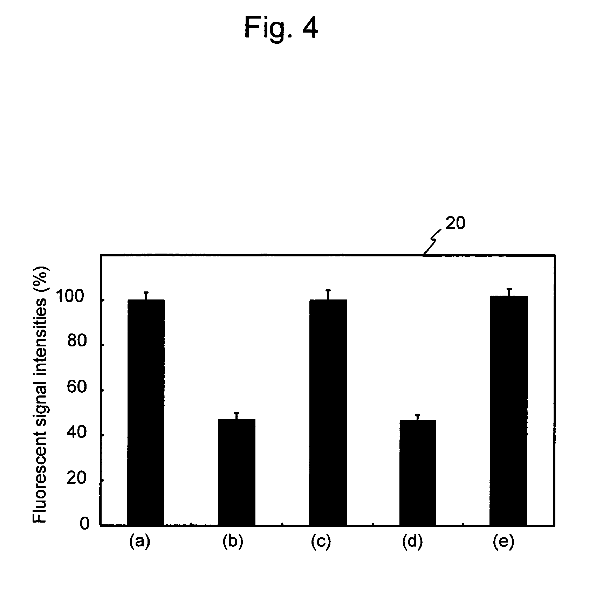

A graph 20 of FIG. 4 shows the comparison of, between using the first and second probes of the present invention and using a Quenching probe, results of measuring fluorescent intensities before and after the PCR amplification reaction of the target nucleic acid. The ordinate of the graph 20 represents fluorescent signal intensities (%) normalized to fluorescent intensities before amplification reaction. The bars (a) to (e) respectively represent fluorescent signal intensity derived from the Quenching probe or the first probe according to the present invention before or after amplification reaction. The bar (a) represents fluorescent signal intensity from the Quenching probe before amplification reaction, and the bar (b) represents fluorescent signal intensity from the Quenching probe after amplification reaction. In the comparison between (a) and (b), the fluorescent signal intensity after amplification reaction is decreased to about 50% of that before amplification reaction. On the other hand, the bar (c) represents fluorescent signal intensity before amplification reaction in the system using the first and second probes, and the bar (d) represents fluorescent signal intensity after amplification reaction in the system using the first and second probes. In the comparison between (c) and (d), the fluorescent signal intensity after amplification reaction is decreased to about 45% of that before amplification reaction. This result demonstrates that the presence or absence of a target nucleic acid can be determined using the first and second probes of the present invention in the same way as in the use of the conventional Quenching probe. Moreover, the bar (e) represents, as a control, fluorescent signal intensity after amplification reaction without the second probe (using only the first probe). In the comparison between (c) and (e), the fluorescent signal intensity was hardly changed before and after amplification reaction. This result demonstrates that the function of the nucleic acid detection probe of the present invention requires using the second probe labeled with a quencher. Thus, it can be understood that the presence or absence of a target nucleic acid can be determined using the first and second probes of the present invention.

In general, the ratio between fluorescent intensity in the absence of a target nucleic acid and fluorescent intensity in the presence of a target nucleic acid serves as an index for showing the sensitivity of a detection probe. In the present invention, fluorescent intensity is large in the absence of the target nucleic acid, and decrease in fluorescent signal through fluorescent energy transfer is observed in the presence of the target nucleic acid. Thus, the fluorescent intensity ratio between before decrease of fluorescent intensity immediately after the initiation of reaction and after decrease of fluorescent intensity is defined as a quenching rate that serves as an index for showing a decrement in fluorescent signal. A probe with a high quenching rate can be determined as a probe excellent in detection sensitivity.

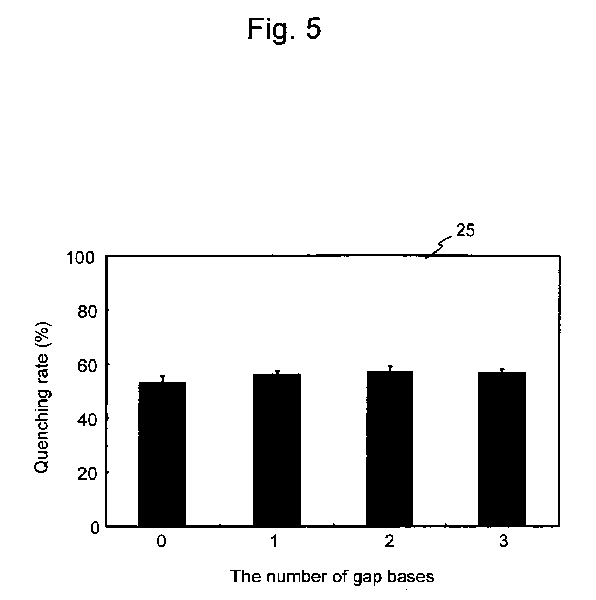

FIG. 5 is a graph showing the relationship of a quenching rate with the number of gap bases between the first and second probes. A graph 25 is a plot with the number of gap bases between the first and second probes as an abscissa against the quenching rate (%) as an ordinate. From the graph 25, it is evident that the quenching rate exhibits the maximum value when the number of gap bases is 2, and however, this maximum value is almost the same as that when the number of gap bases is 1 or 3. Thus, it is evident that the number of gap bases according to the present invention is on the order of 0 to 10 bases, preferably 0 to 3 bases, more preferably 1 base.

In the present invention, the first and second probes need to simultaneously hybridize to the target nucleic acid. This is because even if only the first probe, or only the second probe, hybridizes to the target nucleic acid, quenching effects are not observed, resulting in unsuccessful determination of the presence or absence of the target nucleic acid. The simultaneous hybridization of the first and second probes to the target nucleic acid requires adjusting the Tm values of the first and second probes to as equal values as possible.

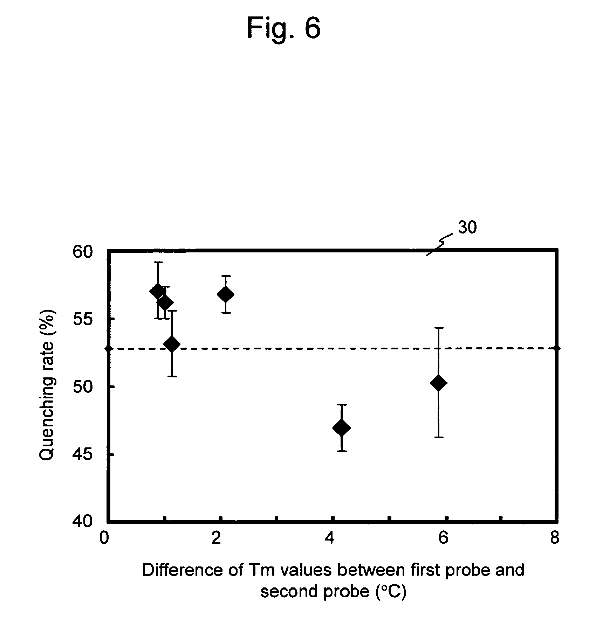

FIG. 6 shows the relationship of the difference of Tm values between the first and second probes with a quenching rate (%). A graph 30 is a plot with the difference of Tm values between the first and second probes as an abscissa against the quenching rate (%) as an ordinate. The line of a quenching rate of 52.8% indicated by a dotted line in the graph represents an average quenching rate obtained from three measurements using the conventional Quenching probe. When the difference of Tm values between the two probes is up to around 2°C, the quenching rate exceeds that of the conventional Quenching probe. However, as the difference of Tm values is as large as 4°C or 6°C, the quenching rate gets smaller than that of the Quenching probe, demonstrating decrease in probe sensitivity. Moreover, as the difference of Tm values gets larger, the quenching rate also varies greatly, demonstrating reduction in the reproducibility of probe performance. From this result, the difference of Tm values between the two probes is preferably within 3°C.

Alternatively, the presence or absence of the target nucleic acid can also be determined in real time by measuring fluorescent intensity during amplification reaction. From the real-time measurement results, the target nucleic acid can also be quantified.

### Examples

Hereinafter, the present invention will be described with reference to Examples. However, the present invention is not intended to be limited to these Examples.

### [Example 1]

### 1. Materials

### 1) Primers used in Example 1

For LAMP reaction, forward inner primer:
5'-tcc agg ggt ctt aac ttg atg gaa aaa t-3' (SEQ ID NO: 1)

For LAMP reaction, forward outer primer:
5'-tcc aga aac gtt tcg-3' (SEQ ID NO: 2)

For LAMP reaction, reverse inner primer:
5'-gga tcc agg ccc aga aaa aaa gac tgt-3' (SEQ ID NO: 3)

For LAMP reaction, reverse outer primer:
5'-agg gct tgg tca ata t-3' (SEQ ID NO: 4)

### 2) Oligonucleotide probes used in Example 1

First detection probe:
5'-ggt ttc tca gga agc aaa aaa ctt ggc ctt cct gg-(BHQ-1)-3' (SEQ ID NO: 5)
Second detection probe:
5'-(FAM)-tcc agg ggg tgc tta caa tcc tga tgt ttt cat tc-(P)-3' (SEQ ID NO: 6)

### 3) Composition of reaction solution used in Example 1 (values in parentheses represent final concentrations)

Tris-HCl pH 8.2 (10 mM), KCl (20 mM), (NH₄)₂SO₄ (5 mM), MgSO₄ (2 mM), dATP (0.25 mM), dCTP (0.25 mM), dGTP (0.25 mM), dTTP (0.25 mM), Triton X-100 (0.05%), Betaine (250 mM)

### 4) Composition of enzyme used in Example 1

Bst DNA polymerase 8 U

### 2. Method

To confirm whether a target nucleic acid can be detected using nucleic acid detection probes of the present invention described in FIG. 1, LAMP reaction was performed, and the amplification products were detected using a fluorescence plate reader. The LAMP reaction was performed using 100 ng of human genomic DNA as a template, and the forward inner and outer primers, the reverse inner and outer primers, and the Bst DNA polymerase described in the paragraph 1. to amplify a cytochrome P450 gene.

The first detection probe is labeled at the 3' end with a quencher BHQ-1 and has a sequence hybridizing to the cytochrome P450 gene as a target nucleic acid. Moreover, the second detection probe is labeled at the 5' end with a fluorophore FAM and phosphorylated at the 3' end to prevent the probe from being extended. The second detection probe also has a sequence hybridizing to the cytochrome P450 gene and is designed to hybridize thereto to give a gap of 1 base downstream of the first detection probe.

The human genomic DNA as a template was added to the reaction solution containing the primers, the oligonucleotide probes, and the Bst DNA polymerase described in the paragraph 1., and the solution was incubated for 90 minutes in a heat block set to 60°C. Then, the fluorescent signal of the reaction solution was measured using the fluorescence plate reader.

### 3. Results

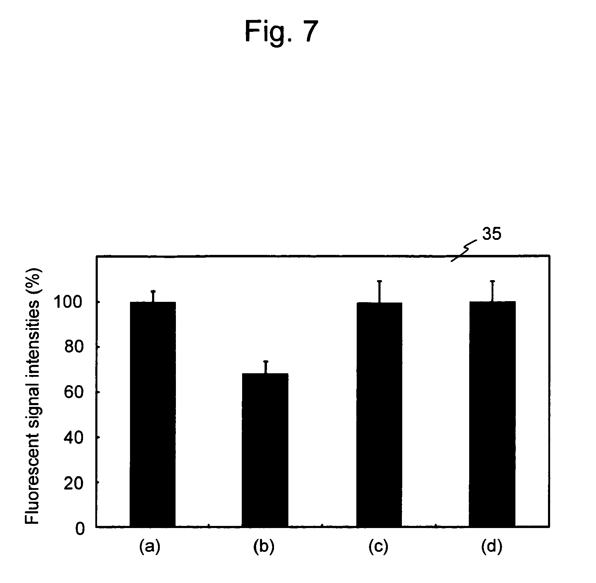

The measurement results are shown in FIG. 7. A graph 35 is a plot of fluorescent signal intensities (%) of the reaction solution under various conditions. The bars (a) and (c) in the graph 35 respectively represent fluorescent signal intensity before reaction, and the bars (b) and (d) respectively represent fluorescent signal intensity after the completion of reaction. The fluorescent signal intensity after nucleic acid amplification reaction represented by (b) was normalized with the fluorescent signal intensity of (a) as 100%. The reaction solutions of (a) and (b) contain the template DNA, whereas the reaction solutions of (c) and (d) serve as a negative control free from the template DNA. From the fluorescent signals of (a) and (c), it could be confirmed that the fluorescent signal intensities before amplification reaction are almost equally high regardless of the presence or absence of the template DNA. In the reaction solution of (b) containing the template DNA, the fluorescent signal intensity was decreased as the reaction progressed. In the reaction solution of (d) as a negative control free from the template DNA, the fluorescent signal intensity was not decreased even as the reaction progressed. From these results, it could be confirmed that quenching occurs by the effects of the probes of the present invention, leading to successful detection of the target nucleic acid.

### [Example 2]

### 1. Materials

### 1) Primers used in Example 2

For PCR reaction, forward primer:
5'-gtc cca tta ttt tcc aga aac gtt tcg-3' (SEQ ID NO: 7)

For PCR reaction, reverse primer:
5'-aag tac ttc agg gct tgg tca ata t-3' (SEQ ID NO: 8)

### 2) Oligonucleotide probes used in Example 2

First detection probe:
5'-ggt ttc tca gga agc aaa aaa ctt ggc ctt acc tgg-(FAM)-3' (SEQ ID NO: 9)
Second detection probe:
5'-(BHQ-1)-tcc agg ggg tgc tta caa tcc tga tgt ttt cat tc-(NH₂)-3' (SEQ ID NO: 10)

### 3) Composition of reaction solution used in Example 2 (values in parentheses represent final concentrations)

Tricine-KOH pH 8.0 (40 mM), KCl (16 mM), MgSO₄ (3.5 mM), dATP (0.4 mM), dCTP (0.4 mM), dGTP (0.4 mM), dTTP (0.4 mM), BSA (3.75 mg/mL)

### 4) Composition of enzyme used in Example 2

TITANIUM Taq polymerase 2 U (Clontech)

### 2. Method

To confirm whether a target nucleic acid can be detected using nucleic acid detection probes of the present invention described in FIG. 2, PCR reaction was performed, and the amplification products were detected using a real-time detector. The PCR reaction was performed using 10² to 10⁸ copies of human genomic DNA as a template, and the PCR reaction forward and reverse primers and the TITANIUM Taq polymerase (Clontech) described in the paragraph 1. to amplify a cytochrome P450 gene.

In this context, the TITANIUM Taq polymerase (Clontech) is an N-terminal deletion mutant of Taq polymerase, which is deficient in 5'→3' exonuclease activity due to this deletion. Therefore, the first and second detection probes contained in the reaction solution hybridize without degradation to the amplification product. As a result, fluorescence is quenched through the fluorescent resonance energy transfer between the probes. The first detection probe is labeled at the 3' end with a fluorophore FAM and has a sequence hybridizing to the cytochrome P450 gene as a target nucleic acid. Moreover, the second detection probe is labeled at the 5' end with a quencher BHQ-1 and aminated at the 3' end to prevent the probe from being extended. This probe also has a sequence hybridizing to the cytochrome P450 gene and is designed to hybridize thereto to give a gap of 1 base downstream of the first detection probe.

The human genomic DNA as a template was added to the reaction solution containing the primers, the oligonucleotide probes, and the TITANIUM Taq polymerase described in the paragraph 1. Changes in the fluorescent signal intensity of the reaction solution were measured over time using the real-time PCR detector set to perform 60 thermal cycles each involving steps at 95°C for 20 seconds and at 55°C for 40 seconds.

### 3. Results

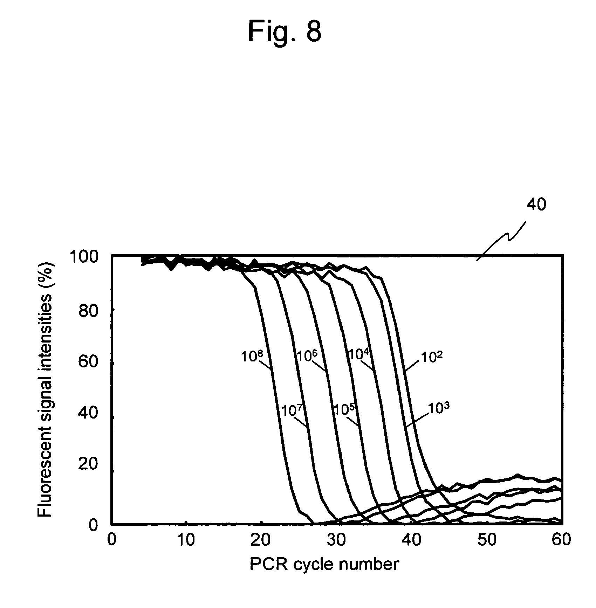

The measurement results are shown in a graph 40 in FIG. 8. The abscissa represents a cycle number, and the ordinate represents the fluorescent signal intensities of FAM. The fluorescent signal intensities are normalized with fluorescent intensity before nucleic acid amplification as 100% and that after nucleic acid amplification as 0%. In the graph, the fluorescent signal intensity of FAM at the initial template concentration of 10⁸ copies started to decrease around the 20th cycle after the initiation of reaction. This decrease in the fluorescent signal intensity of FAM shows that the first and second detection probes hybridize to the amplification product of the cytochrome P450 gene such that fluorescence is quenched. The cycle number at which the fluorescent signal intensity started to decrease got larger dependently on the initial template concentration. Therefore, it could be confirmed that the target nucleic acid can be detected in real time using the probes of the present invention.

### [Example 3]

### 1. Materials

### 1) Primers used in Example 3

For PCR reaction, forward primer:
5'-gca gaa agc gtc tag cca tgg cg-3' (SEQ ID NO: 11)

For PCR reaction, reverse primer:
5'-cat ggt gca cgg tct acg aga cc-3' (SEQ ID NO: 12)

Reverse transcription primer:
5'-tgc tca tgg tgc acg gtc ta-3' (SEQ ID NO: 13)

### 2) Oligonucleotide probe used in Example 3

5'-(FAM)-tgtt ggg tcgcg aaag gcc ccttc tca ctg ttc tct cat aga agt gat gag gga aca gag cac tca tct ctt ctc cct gtt aga ctg cta gcc gag tag-(BHQ-1)-3' (SEQ ID NO: 14)

The underlined parts hybridize to a template.

### 3) Composition of reaction solution used in Example 3

In Example 3, Gene Taq Universal Buffer (Nippon Gene Co., Ltd.) was used as a reaction solution. The reaction solution prepared contained dATP (0.2 mM), dCTP (0.2 mM), dGTP (0.2 mM), and dTTP (0.2 mM).

### 4) Composition of enzyme used in Example 3

Recombinant Taq DNA Polymerase: Gene Taq 0.625 U (Nippon Gene Co., Ltd.)

### 2. Method

To confirm whether a target nucleic acid can be detected using a nucleic acid detection probe of the present invention described in FIG. 3, PCR reaction was performed, and the amplification products were detected using a fluorescence plate reader. The PCR reaction was performed using, as a template, 1 ng of cDNA obtained by reverse transcription of HCV genomic RNA with the reverse transcription primer, and the PCR reaction forward and reverse primers and the Gene Taq (Nippon Gene Co., Ltd.) described in the paragraph 1. to amplify a sequence of interest.

In this context, the Gene Taq (Nippon Gene Co., Ltd.) is deficient in endogenous 5'→3' exonuclease activity due to the N-terminal partial deletion of wild-type Taq DNA polymerase. Therefore, the detection probe contained in the reaction solution hybridizes without degradation to the amplification product. As a result, fluorescence is quenched through the fluorescent resonance energy transfer between the labeled ends of the probe. The detection probe is labeled at the 3' end with a quencher BHQ-1 and at the 5' end with a fluorophore FAM. The detection probe is designed such that the 18-base sequence (gat gag ccg atc gtc aga: SEQ ID NO: 15) from the 3' end and the 19-base sequence (tgt tgg gtc gcg aaa ggc c: SEQ ID NO: 16) from the 5' end hybridize to the HCV gene in tandem to give a gap of 1 base.

The cDNA as a template was added to the reaction solution containing the primers, the oligonucleotide probe, and the Gene Taq described in the paragraph 1., and subjected to 40 thermal cycles each involving steps at 95°C for 20 seconds and at 55°C for 40 seconds. Then, the fluorescent signal of the reaction solution was measured using the fluorescence plate reader.

### 3. Results

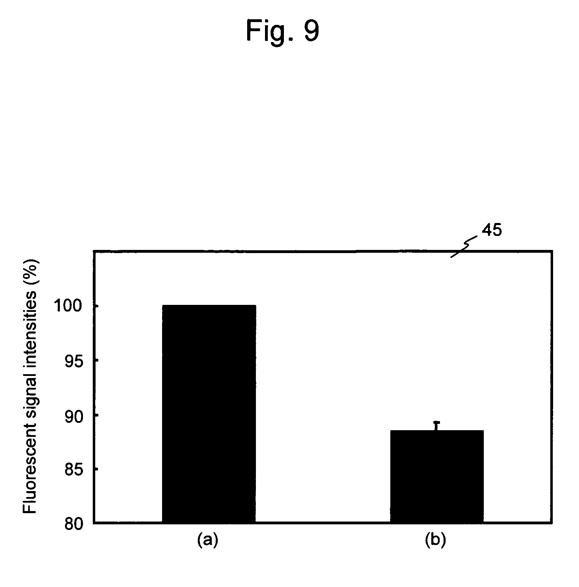

The measurement results are shown in a graph 45 in FIG. 9. The graph 45 is a plot of fluorescent signal intensities (%) of the reaction solution. The bar (a) in the graph 45 represents fluorescent signal intensity before reaction, and the bar (b) represents fluorescent signal intensity after the completion of reaction. The fluorescent signal intensity after nucleic acid amplification reaction represented by (b) was normalized with the fluorescent signal intensity of (a) as 100%. From this result, it could be confirmed that quenching occurs by the effects of the probe of the present invention, leading to successful detection of the target nucleic acid.

### [Example 4]

### 1. Materials

### 1) Primers used in Example 4

For PCR reaction, forward primer:
5'-tcttt ccaag caaca gcag-3' (SEQ ID NO: 17)

For PCR reaction, reverse primer:
5'-cgttt gctga tgaac taagt c-3' (SEQ ID NO: 18)

### 2) Oligonucleotide probes used in Example 4

First detection probes:
5'-caatg accaa atcaa agaaa t-(BHQ-0)-3' (SEQ ID NO: 19)
5'-caatg accaa atcaa agaaa t-(DABCYL)-3' (SEQ ID NO: 20)

Second detection probes:
5'-(ALEXA 350)-actcg caagg ttagt gctga g-(NH₂)-3' (SEQ ID NO: 21)
5'-(ALEXA 790)-actcg caagg ttagt gctga g-(NH₂)-3' (SEQ ID NO: 22)

### 3) Composition of reaction solution used in Example 4

In Example 4, Gene Taq Universal Buffer (Nippon Gene Co., Ltd.) was used as a reaction solution. The reaction solution prepared contained dATP (0.2 mM), dCTP (0.2 mM), dGTP (0.2 mM), and dTTP (0.2 mM).

### 4) Composition of enzyme used in Example 4

Recombinant Taq DNA Polymerase: Gene Taq 0.625 U (Nippon Gene Co., Ltd.)

### 2. Method

To confirm whether a target nucleic acid can be detected using nucleic acid detection probes of the present invention described in FIG. 1, PCR reaction was performed, and the amplification products were detected using a fluorescence plate reader. The PCR reaction was performed using the genomic DNA of bacteriophage phi X174 as a template, and the PCR reaction forward and reverse primers and the Gene Taq (Nippon Gene Co., Ltd.) described in the paragraph 1. to amplify a sequence of interest.

In this context, the Gene Taq (Nippon Gene Co., Ltd.) is deficient in endogenous 5'→3' exonuclease activity due to the N-terminal partial deletion of wild-type Taq DNA polymerase. Therefore, the detection probes contained in the reaction solution hybridize without degradation to the amplification product. As a result, fluorescence is quenched through the fluorescent resonance energy transfer between the probes.

The first detection probe represented by SEQ ID NO: 19 is labeled at the 3' end with a quencher BHQ-0 and has a sequence hybridizing to the bacteriophage phi X174 genome as a target nucleic acid. Moreover, the second detection probe represented by SEQ ID NO: 21 is labeled at the 5' end with a fluorophore ALEXA 350 and aminated at the 3' end to prevent the probe from being extended. The second detection probe represented by SEQ ID NO: 21 also has a sequence hybridizing to the bacteriophage phi X174 genome and is designed to hybridize thereto to give a gap of 1 base downstream of the first detection probe represented by SEQ ID NO: 19.

Moreover, the first detection probe represented by SEQ ID NO: 20 has the same nucleotide sequence as that of the first detection probe represented by SEQ ID NO: 19 and is labeled at the 3' end with a quencher DABCYL. The second detection probe represented by SEQ ID NO: 22 has the same nucleotide sequence as that of the second detection probe represented by SEQ ID NO: 21. The second detection probe represented by SEQ ID NO: 22 is labeled at the 5' end with a fluorophore ALEXA 790 and aminated at the 3' end to prevent the probe from being extended. When the first detection probe represented by SEQ ID NO: 20 and the second detection probe represented by SEQ ID NO: 22 are used in combination, the second detection probe hybridizes to the bacteriophage phi X174 genome to give a gap of 1 base downstream of the first detection probe, in the same way as in the use of the first detection probe represented by SEQ ID NO: 19 and the second detection probe represented by SEQ ID NO: 21 in combination.

The genomic DNA as a template was added to the reaction solution containing the primers, the first and second detection probes (combination of SEQ ID NOs: 19 and 21 or combination of SEQ ID NOs: 20 and 22), and the Gene Taq described in the paragraph 1., and subjected to 45 thermal cycles each involving steps at 95°C for 15 seconds and at 60°C for 60 seconds. Then, the fluorescent signal of the reaction solution was measured using the fluorophotometer.

### 3. Results

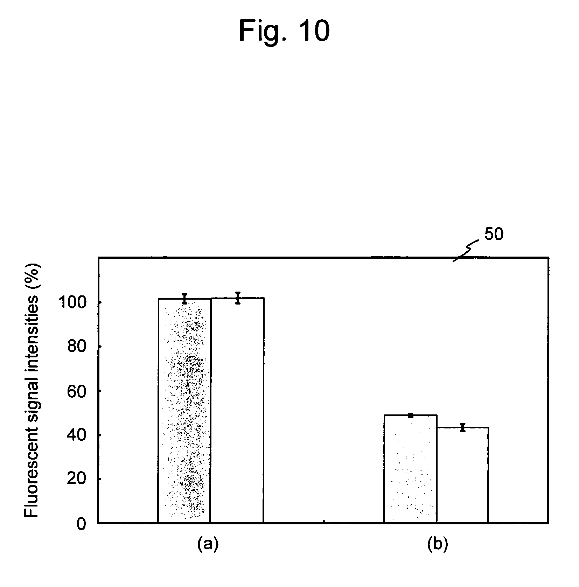

The measurement results are shown in a graph 50 in FIG. 10. The graph 50 is a plot of fluorescent signal intensities (%) of the reaction solution. The bar (a) in the graph 50 represents fluorescent signal intensity before reaction, and the bar (b) represents fluorescent signal intensity after the completion of reaction. The fluorescent signal intensity after nucleic acid amplification reaction represented by (b) was normalized with the fluorescent signal intensity of (a) as 100%. In the graph, the bars indicated in gray represent the fluorescent intensities before reaction and after the completion of reaction of the reaction solution using the combination of SEQ ID NOs: 19 and 21, wherein the fluorescent intensities in the wavelength range of 400 to 500 nm were measured after excitation at an excitation wavelength of 345 nm using the fluorophotometer. On the other hand, the bars indicated in white represent the fluorescent intensities before reaction and after the completion of reaction of the reaction solution using the combination of SEQ ID NOs: 20 and 22, wherein the fluorescent intensities in the wavelength range of 800 to 850 nm were measured after excitation at an excitation wavelength of 790 nm using the fluorophotometer. Both the gray and white bars show that quenching occurred such that the fluorescent intensity after the completion of reaction was decreased to less than half the fluorescent intensity before reaction. From these results, it could be confirmed that a fluorophore such as ALEXA 350, which can be excited with ultraviolet light, and a fluorophore such as ALEXA 790, which is a fluorescent dye in a far infrared region, can be used in the present invention.

### [Example 5]

1) Primers used in Example 5
   PCR reaction sense primer:
      5'-tgcta tgtca cttcc ccttg gttct ctca-3' (SEQ ID NO: 23)
   PCR reaction antisense primer:
      5'-agtgg gggga catca agcag ccatg caaa-3' (SEQ ID NO: 24)
2) Oligonucleotide probes used in Example 5
   First detection probes:
      5'-ctgcagaatgggacaggit-(DABCYL)-3' (SEQ ID NO: 25)
      5'-gaggaagctgcagaatgg-(DABCYL)-3' (SEQ ID NO: 26)
      5'-aagataccatcaatgagg-(DABCYL)-3' (SEQ ID NO: 27)
      5'-cagaatgggatagggt-(DABCYL)-3' (SEQ ID NO: 28)
   Second detection probes:
      5'-(ALEXA 350)-acagccagtacatgcag-(NH₂)-3' (SEQ ID NO: 29)
      5'-(ALEXA 430)-gatagattgcatcccagt-(NH₂)-3' (SEQ ID NO: 30)
      5'-(ALEXA 594)-aggctgcagaatggga-(NH₂)-3' (SEQ ID NO: 31)
      5'-(ALEXA 790)-acacccagtacatgcagggc-(NH₂)-3' (SEQ ID NO: 32)
3) Composition of reaction solution used in Example 5 (values in parentheses represent final concentrations)

Tricine-KOH pH 8.0 (40 mM), KCl (16 mM), MgSO₄ (3.5 mM), dATP (0.4 mM), dCTP (0.4 mM), dGTP (0.4 mM), dTTP (0.4 mM), BSA (3.75 mg/mL)

### 4) Composition of enzyme used in Example 5

TITANIUM Taq polymerase 2 U (Clontech)

### 2. Method

To confirm whether a target nucleic acid can be genotyped using nucleic acid detection probes of the present invention described in FIG. 1, PCR reaction was performed, and the amplification products were detected using a real-time detector. The PCR reaction was performed using the genomic RNA of HIV-1 virus as a template, and the PCR reaction sense and antisense primers and the TITANIUM Taq polymerase (Clontech) described in the paragraph 1. to amplify a *gag* region.

In this context, the TITANIUM Taq polymerase (Clontech) is an N-terminal deletion mutant of Taq polymerase, which is deficient in 5'→3' exonuclease activity due to this deletion. Therefore, the first and second detection probes contained in the reaction solution hybridize without degradation to the amplification product. As a result, fluorescence is quenched through the fluorescent resonance energy transfer between the probes.

The first detection probes represented by SEQ ID NOs: 25, 26, 27, and 28 are labeled at the 3' end with a quencher DABCYL and have sequences hybridizing to the *gag* region as a target nucleic acid in subtypes A, B, C, and CRF (Circulating Recombinant form) AE, respectively, of the HIV-1 virus genome. Moreover, the second detection probes represented by SEQ ID NOs: 29, 30, 31, and 32 are labeled at the 5' end with fluorophores ALEXA 350, ALEXA 430, ALEXA 594, and ALEXA 790, respectively and aminated at the 3' end to prevent the probe from being extended. These second probes also have sequences hybridizing to the *gag* region as a target nucleic acid in subtypes A, B, C, and CRF (Circulating Recombinant form) AE, respectively, of the HIV-1 virus genome and are designed to hybridize thereto downstream of the first detection probe hybridizing to the same subtype. Specifically, to the subtype A, the second detection probe of SEQ ID NO: 29 hybridizes downstream of the first detection probe of SEQ ID NO: 25; to the subtype B, the second detection probe of SEQ ID NO: 30 hybridizes downstream of the first detection probe of SEQ ID NO: 26; to the subtype C, the second detection probe of SEQ ID NO: 31 hybridizes downstream of the first detection probe of SEQ ID NO: 27; and to the subtype CRF AE, the second detection probe of SEQ ID NO: 32 hybridizes downstream of the first detection probe of SEQ ID NO: 28.

The HIV-1 virus genome as a template was added to the reaction solution containing the primers, the first and second detection probes (combination of SEQ ID NOs: 25 and 29, combination of SEQ ID NOs: 26 and 30, combination of SEQ ID NOs: 27 and 31, or combination of SEQ ID NOs: 28 and 32), and the TITANIUM Taq polymerase described in the paragraph 1., and subjected to PCR under conditions of 40 thermal cycles each involving steps at 95°C for 20 seconds and at 55°C for 40 seconds. Fluorescent intensities at 55°C respectively obtained by excitation with a white light source were measured every cycle through a band-pass filter which exhibited a passband of 30-nm bandwidth at a center wavelength of 440 nm (for ALEXA 350), 30-nm bandwidth at a center wavelength of 540 nm (for ALEXA 430), 30-nm bandwidth at a center wavelength of 620 nm (for ALEXA 594), or 30-nm bandwidth at a center wavelength of 810 nm (for ALEXA 790).

### 3. Results

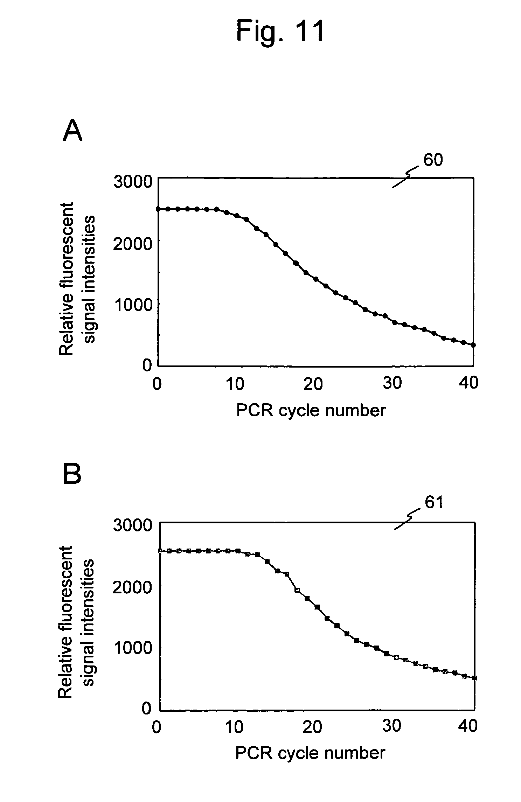

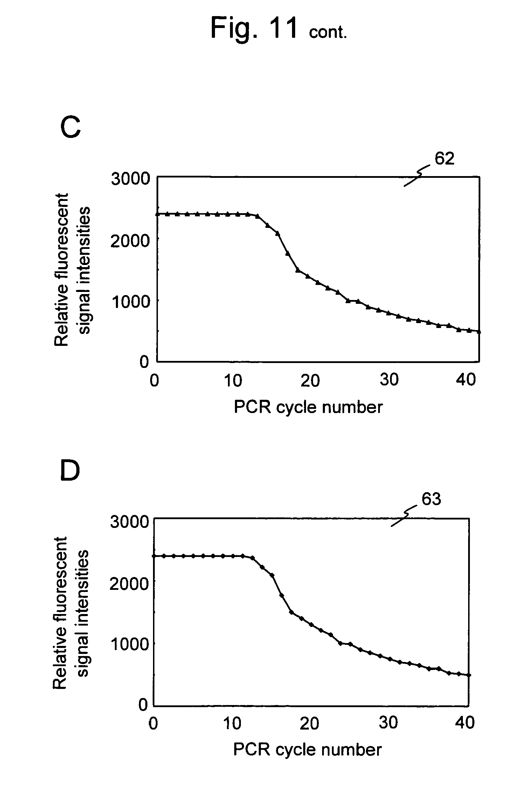

The measurement results are shown in FIG. 11. All graphs of FIG. 11 show a PCR cycle number in the abscissa and relative fluorescent signal intensities in the ordinate. FIG. 11A (graph 60) shows results of detecting the HIV-1 subtype A using the combination of SEQ ID NOs: 25 and 29; FIG. 11B (graph 61) shows results of detecting the HIV-1 subtype B using the combination of SEQ ID NOs: 26 and 30; FIG. 11C (graph 62) shows results of detecting the HIV-1 subtype C using the combination of SEQ ID NOs: 27 and 31; and FIG. 11D (graph 63) shows results of detecting the HIV-1 subtype CRF AE using the combination of SEQ ID NOs: 28 and 32. In all the graphs A to D, the fluorescent signal intensity started to decrease at the 10th or later cycles. This decrease in the fluorescent signal intensity shows that the first and second detection probes hybridize to the amplification product of the genomic RNA of HIV-1 virus such that fluorescence is quenched. The fluorescent wavelengths of these four fluorophores are 440 nm for ALEXA 350, 540 nm for ALEXA 430, 620 nm for ALEXA 594, and 810 nm for ALEXA 790, which differ from one another by at least 80 nm or more. Therefore, it could be confirmed that fluorescence detection is achieved in a state with less influence of leakage of fluorescence derived from the fluorophores into one another. In a conventional DNA sequencer, the fluorescent wavelengths of four fluorophores differ from one another only by approximately 20 nm. However, even for this difference of approximately 20 nm, genotyping can be achieved in consideration of the influence of leakage of fluorescence derived from the fluorophores into one another. This demonstrates that using the detection probes of the present invention, the multiplex detection of 10 or more target nucleic acids can be performed in combination of fluorophores having fluorescent wavelengths in the range of 440 to 800 nm.

According to the present invention, the presence or absence of a target nucleic acid can be determined based on changes in the fluorescent intensity of a reaction solution attributed to the hybridization of two oligonucleotide probes thereto. Moreover, the oligonucleotide probes according to the present invention are designed with a high degree of flexibility and are applicable to various fluorophores. Therefore, the oligonucleotide probes according to the present invention facilitate real-time multiplex detection during the amplification reaction of trace amounts of nucleic acids, wherein plural kinds of target nucleic acids are simultaneously detected using plural fluorophores. Thus, the present invention is useful in, for example, medical or life science fields such as gene diagnosis, which require amplifying trace amounts of nucleic acids.

### [Free Text of Sequence Listing]

SEQ ID NO: 1 - Description of Artificial Sequence: forward inner amplification primer used in the present invention
SEQ ID NO: 2 - Description of Artificial Sequence: forward outer amplification primer used in the present invention
SEQ ID NO: 3 - Description of Artificial Sequence: reverse inner amplification primer used in the present invention
SEQ ID NO: 4 - Description of Artificial Sequence: reverse outer amplification primer used in the present invention
SEQ ID NO: 5 - Description of Artificial Sequence: first detection oligonucleotide probe used in the present invention
SEQ ID NO: 6 - Description of Artificial Sequence: second detection oligonucleotide probe used in the present invention
SEQ ID NO: 7 - Description of Artificial Sequence: forward primer used in the present invention
SEQ ID NO: 8 - Description of Artificial Sequence: reverse primer used in the present invention
SEQ ID NO: 9 - Description of Artificial Sequence: first detection oligonucleotide probe used in the present invention
SEQ ID NO: 10 - Description of Artificial Sequence: second detection oligonucleotide probe used in the present invention
SEQ ID NO: 11 - Description of Artificial Sequence: forward primer used in the present invention
SEQ ID NO: 12 - Description of Artificial Sequence: reverse primer used in the present invention
SEQ ID NO: 13 - Description of Artificial Sequence: reverse transcription primer used in the present invention
SEQ ID NO: 14 - Description of Artificial Sequence: detection oligonucleotide probe used in the present invention
SEQ ID NO: 15 - sequence at 3' end hybridizing to HCV gene
SEQ ID NO: 16 - sequence at 5' end hybridizing to HCV gene
SEQ ID NO: 17 - Description of Artificial Sequence: forward primer used in the present invention
SEQ ID NO: 18 - Description of Artificial Sequence: reverse primer used in the present invention
SEQ ID NO: 19 - Description of Artificial Sequence: first detection oligonucleotide probe used in the present invention
SEQ ID NO: 20 - Description of Artificial Sequence: first detection oligonucleotide probe used in the present invention
SEQ ID NO: 21 - Description of Artificial Sequence: second detection oligonucleotide probe used in the present invention
SEQ ID NO: 22 - Description of Artificial Sequence: second detection oligonucleotide probe used in the present invention
SEQ ID NO: 23 - Description of Artificial Sequence: sense primer used in the present invention
SEQ ID NO: 24 - Description of Artificial Sequence: antisense primer used in the present invention
SEQ ID NO: 25 - Description of Artificial Sequence: first detection oligonucleotide probe used in the present invention
SEQ ID NO: 26 - Description of Artificial Sequence: first detection oligonucleotide probe used in the present invention
SEQ ID NO: 27 - Description of Artificial Sequence: first detection oligonucleotide probe used in the present invention
SEQ ID NO: 28 - Description of Artificial Sequence: first detection oligonucleotide probe used in the present invention
SEQ ID NO: 29 - Description of Artificial Sequence: second detection oligonucleotide probe used in the present invention
SEQ ID NO: 30 - Description of Artificial Sequence: second detection oligonucleotide probe used in the present invention
SEQ ID NO: 31 - Description of Artificial Sequence: second detection oligonucleotide probe used in the present invention
SEQ ID NO: 32 - Description of Artificial Sequence: second detection oligonucleotide probe used in the present invention

## Claims

1. A nucleic acid analysis kit comprising first and second probes which hybridize to the same target nucleic acid, wherein
the first probe hybridizes to a region closer to the 3' end of the target nucleic acid than a region to which the second probe hybridizes on the target nucleic acid, and has a fluorophore or quencher at the 3' end, and
the second probe has a quencher at the 5' end when the first probe has a fluorophore at the 3' end, or has a fluorophore at the 5' end when the first probe has a quencher at the 3' end.

2. The kit according to claim 1, wherein the region to which the first probe hybridizes and the region to which the second probe hybridizes are 0 to 10 bases apart on the target nucleic acid.

3. The kit according to claim 1 or 2, wherein the region to which the first probe hybridizes and the region to which the second probe hybridizes are 0 to 3 bases apart on the target nucleic acid.

4. The kit according to any one of claims 1 to 3, wherein the first and second probes differ in melting temperature (Tm value) by 3°C or lower.

5. A nucleic acid analysis method comprising the following steps:
1) detecting, in first detection, the fluorescence of a reaction solution comprising first and second probes which hybridize to the same target nucleic acid;
2) hybridizing the first and second probes to the target nucleic acid;
3) detecting, in second detection, the fluorescence of the reaction solution after the hybridization step; and
4) analyzing the amount of the target nucleic acid by comparing the results of the first and second detections, wherein
the first probe hybridizes to a region closer to the 3' end of the target nucleic acid than a region to which the second probe hybridizes on the target nucleic acid, and has a fluorophore or quencher at the 3' end, and the second probe has a quencher at the 5' end when the first probe has a fluorophore at the 3' end, or has a fluorophore at the 5' end when the first probe has a quencher at the 3' end.

6. The nucleic acid analysis method according to claim 5, wherein the region to which the first probe hybridizes and the region to which the second probe hybridizes are 0 to 10 bases apart on the target nucleic acid.

7. The nucleic acid analysis method according to claim 5 or 6, wherein the region to which the first probe hybridizes and the region to which the second probe hybridizes are 0 to 3 bases apart on the target nucleic acid.

8. The nucleic acid analysis method according to any one of claims 5 to 7, wherein the first and second probes differ in melting temperature (Tm value) by 3°C or lower.

9. A nucleic acid analysis kit comprising a probe having a structure in which first and second sequences which hybridize to the same target nucleic acid are ligated via a third sequence which does not hybridize to the target nucleic acid, wherein
the first sequence hybridizes to a region closer to the 3' end of the target nucleic acid than a region to which the second sequence hybridizes on the target nucleic acid, and has a fluorophore or quencher at the 3' end, and
the second sequence has a quencher at the 5' end when the first sequence has a fluorophore at the 3' end, or has a fluorophore at the 5' end when the first sequence has a quencher at the 3' end.

10. The kit according to claim 9, wherein the region to which the first sequence hybridizes and the region to which the second sequence hybridizes are 0 to 10 bases apart on the target nucleic acid.

11. The kit according to claim 9 or 10, wherein the region to which the first sequence hybridizes and the region to which the second sequence hybridizes are 0 to 3 bases apart on the target nucleic acid.

12. The kit according to any one of claims 9 to 11, wherein the first and second sequences differ in melting temperature (Tm value) by 3°C or lower.

13. A nucleic acid analysis method comprising the following steps:
1) detecting, in first detection, the fluorescence of a reaction solution comprising a probe labeled at one end with a fluorophore and at the other end with a quencher;
2) hybridizing the probe to a target nucleic acid;
3) detecting, in second detection, the fluorescence of the reaction solution after the hybridization step; and
4) analyzing the amount of the target nucleic acid by comparing the results of the first and second detections, wherein
the probe has a structure in which first and second sequences which hybridize to the same target nucleic acid are ligated via a third sequence which does not hybridize to the target nucleic acid, wherein the first sequence hybridizes to a region closer to the 3' end of the target nucleic acid than a region to which the second sequence hybridizes on the target nucleic acid, and has a fluorophore or quencher at the 3' end, and the second sequence has a quencher at the 5' end when the first sequence has a fluorophore at the 3' end, or has a fluorophore at the 5' end when the first sequence has a quencher at the 3' end.

14. The nucleic acid analysis method according to claim 13, wherein the region to which the first sequence hybridizes and the region to which the second sequence hybridizes are 0 to 10 bases apart on the target nucleic acid.

15. The nucleic acid analysis method according to claim 13 or 14, wherein the region to which the first sequence hybridizes and the region to which the second sequence hybridizes are 0 to 3 bases apart on the target nucleic acid.

16. The nucleic acid analysis method according to any one of claims 13 to 15, wherein the first and second sequences differ in melting temperature (Tm value) by 3°C or lower.
